# EUROPÄISCHE PATENTSCHRIFT

(11) **EP 2 190 343 B1**
(45) Veröffentlichungstag und Bekanntmachung des Hinweises auf die Patenterteilung: **22.11.2017**
(21) Anmeldenummer: 08785776.9
(22) Anmeldetag: 30.08.2008
(51) Int. Cl.: A61B 3/00, A61B 3/10, A61F 9/008

(54) **ENERGIESPARENDES MEDIZINISCHES GERÄT**
ENERGY-SAVING MEDICAL DEVICE
APPAREIL MÉDICAL À CONSOMMATION D'ÉNERGIE RÉDUITE

(30) Priorität: 04.09.2007 DE 102007041962
(43) Veröffentlichungstag der Anmeldung: 02.06.2010
(73) Patentinhaber: Carl Zeiss Meditec AG, 07745 Jena (DE)
(72) Erfinder: LÜMKEMANN, Frank, 07751 Jena (DE); WEIGAND, Heino, 4885 Jaggan, QLD (AU)
(74) Vertreter: Loritz, Rainer
(86) Internationale Anmeldenummer: PCT/EP2008/007125
(87) Internationale Veröffentlichungsnummer: WO 2009/030446

(56) Entgegenhaltungen:
- JP-A- 11 249 064
- JP-A- 2001 187 021
- JP-A- 2006 026 000
- US-A- 3 830 562
- US-A- 4 422 457
- US-A- 5 387 952
- US-A- 5 790 234
- US-A1- 2003 025 604
- US-B1- 6 259 486

## Beschreibung

Die Erfindung betrifft ein medizinisches Gerät, bei dem zumindest ein Geräteteil zwischen einem Ruhezustand und einem Betriebszustand umschaltbar ist, aufweisend einen Personendetektor und eine Steuereinheit, die, falls der Personendetektor eine entfernt vom Gerät anwesende Person detektiert, zumindest das Geräteteil in den Betriebszustand schaltet, wobei mittels des Personendetektors eine Person detektierbar ist, die weiter vom Gerät entfernt ist als es für eine Benutzung des Geräts erforderlich ist.

Medizinische Geräte enthalten in der Regel elektrische Bauteile, die im Betriebszustand Energie verbrauchen und dabei Abwärme erzeugen. Die Kühlung solcher Bauteile verbraucht weitere elektrische Energie, führt üblicherweise zur Erwärmung des umgebenden Raums und erzeugt, beispielsweise durch rotierende Lüfter, mehr oder weniger intensiven Lärm.

Medizinische Geräte in einer Praxis eines niedergelassenen Arztes werden typischerweise zu Beginn der Öffnungszeiten der Praxis eingeschaltet und nach Ende der Öffnungszeiten ausgeschaltet, um permanent verfügbar zu sein. In Krankenhäusern sind Medizingeräte aus demselben Grund oft rund um die Uhr eingeschaltet. In beiden Fällen werden die Geräte zwar nicht kontinuierlich benutzt, können aber aufgrund des permanent eingeschalteten Betriebszustandes sowohl wegen der Abwärme als auch wegen des eventuellen Lärms signifikante Gesundheitsbelastungen für das Bedienpersonal sein. Vor allem, wenn mehrere Geräte in einem Raum Abwärme und Lärm erzeugen, erzeugt dies Stress, der allerdings nicht immer bewusst wahrgenommen wird.

Bei anderen medizinischen Geräten ist der Betriebszustand hingegen akustisch und optisch so unauffällig, dass nach dem Ende einer Benutzung, also einer Untersuchung oder einer Behandlung, und der Abfertigung des Patienten leicht übersehen werden kann, dass ein Gerät noch im Betriebszustand ist. Da von solchen Geräten keine unmittelbar fühlbare Belastung des Bedienpersonals ausgeht, besteht möglicherweise für einzelne Personen kein Anlass, das Gerät bis zur nächsten Benutzung auszuschalten.

Es ist jedoch generell erstrebenswert, den Energiebedarf von technischen Geräten so niedrig wie möglich zu halten, um geringe Betriebskosten und ein gutes ökologisches Kosten-Nutzen-Verhältnis zu erreichen. Auch werden medizinische Geräte teilweise mit Akkumulatoren oder Batterien betrieben, sei es im Falle eines Stromausfalls oder in Gebieten ohne elektrisches Netz. Bei solchen Geräten ist es besonders wichtig, dass nicht unnötig Energie verbraucht wird.

Zudem haben die meisten elektrischen oder elektronischen Bauteile nur eine begrenzte Lebensdauer, die üblicherweise in einer mittleren Anzahl von zu erwartenden Betriebsstunden bis zu einem Ausfall (MTTF bzw. MTBF) angegeben wird. Beim Dauerbetrieb eines nur intermittierend benutzten Medizingeräts entstehen daher höhere Betriebskosten als beim Betrieb nach Bedarf. Durch den häufiger erforderlichen Austausch eines defekten Bauteils verschlechtert sich zudem das ökologische Kosten-Nutzen-Verhältnis des Geräts.

JP 2001187021 zeigt einen ophthalmologischen Apparat mit einem Personendetektor, der bei Detektion einer Person einen Energiesparmodus beendet.

Der Erfindung liegt die Aufgabe zugrunde, einen reduzierten Energieverbrauch von medizinischen Geräten zu ermöglichen.

Die Aufgabe wird erfindungsgemäß durch die Merkmalskombinationen des Anspruchs 1 gelöst. Vorteilhafte Ausgestaltungen der Erfindung sind in den abhängigen Ansprüchen angegeben.

Erfindungsgemäß ist der Personendetektor als Bewegungsmelder ausgebildet und kann in einem bestimmten Raumbereich passiv mittels Infrarotstrahlung Bewegungen und/oder Temperaturveränderungen ermitteln, wobei mittels des Personendetektors eine Person detektierbar ist, die mindestens 1 m vom Gerät entfernt ist, wobei die Steuereinheit zunächst ermittelt, ob die detektierte Person sich dem Gerät nähert, und nur bei ermittelter Annäherung in den Betriebszustand schaltet. Eine Vorlauf- oder Aufwärmzeit (nachfolgend vereinheitlicht als Anlaufzeit bezeichnet) eines medizinischen Gerätes aus dem Ruhezustand kann ohne negative Seiteneffekte nicht verkürzt werden. Durch Detektion einer noch entfernten Person kann ein notwendiger Vorlauf beziehungsweise ein notwendiges Aufwärmen jedoch frühzeitig begonnen werden, so dass das Medizingerät bei Ankunft der detektierten Person ohne oder allenfalls mit geringer Verzögerung betriebsbereit ist. Nach der Benutzung kann das Gerät beziehungsweise das Geräteteil wieder in den Ruhezustand versetzt werden. Durch die automatische Betriebsbereitschaft ohne oder mit nur geringer Verzögerung besteht kein Grund mehr, ein Medizingerät permanent im Betriebszustand zu belassen. Die Erfindung reduziert dadurch effektiv den Energieverbrauch des medizinischen Gerätes.

Indem die Steuereinheit zunächst ermittelt, ob die detektierte Person sich dem Gerät nähert, und nur in den Betriebszustand schaltet, wenn eine Annäherung ermittelt werden kann, kann ein unnötiges Einschalten des Betriebszustandes vermieden werden. Beispielsweise wird bei offener Tür im Falle einer eintretenden Person das Gerät in den Betriebszustand versetzt, nicht jedoch bei Personen, die vor der Tür auf dem Flur vorübergehen.

Besonders bevorzugt ist eine Ausführungsform eines erfindungsgemäßen Medizingeräts, bei welcher zusätzlich zu einer personendetektionsbasierten Einschaltung die Steuereinheit nach Ablauf eines vorgebbaren oder vorgegebenen Zeitraums, in dem die Steuereinheit keine Benutzung des Geräts ermittelt und/oder in dem der Personendetektor keine Person detektiert, zumindest das Geräteteil vom Betriebszustand in den Ruhezustand schaltet. Dies reduziert den Energieverbrauch maximal. Das automatische, zeitbasierte Ausschalten vermeidet einen versehentlichen Permanentbetrieb des Geräts. Die begrenzte Lebensdauer kritischer Bauteile wird besser ausgenutzt. Das ökologische Kosten-Nutzen-Verhältnis wird so in mehrfacher Hinsicht verbessert.

Je nach Ausführung des Personendetektors kann bei einer längeren Benutzungspause oder bei einer nicht erfassbaren Form der Benutzung trotz Anwesenheit von Bedienpersonal und Patient ein Nichtbenutzungszeitraum verstreichen. Vorteilhafterweise gibt die Steuereinheit in Ausführungsformen mit zeitbasierter Schaltung in den Ruhezustand zu einem vorgebbaren oder vorgegebenen Vorwarnzeitpunkt vor Ablauf des Zeitraums ein Warnsignal aus. Dies ermöglicht es dem Bedienpersonal, eine kurze, von der Steuereinheit erfassbare Bedienungshandlung ohne weitere Wirkung vorzunehmen oder sich lediglich zu bewegen, so dass der Zeitraum der Nichtbenutzung beendet und die bevorstehende Umschaltung in den Ruhezustand vermieden werden kann.

Vorzugsweise ist mittels des Personendetektors eine Person in einem das Gerät umgebenden architektonischen Innenraum, in einem Raumbereich im Umfeld des Geräts oder an einer bestimmten Stelle detektierbar. Dadurch die Beschränkung des Detektionsbereichs kann ein unnötiges Schalten in den Betriebszustand vermieden werden. Der Energieverbrauch kann so minimiert werden.

Als Bewegungsmelder werden alle Sensoren angesehen, die in einem bestimmten Raumbereich, beispielsweise einem festen Raumwinkel, Bewegungen und/oder Temperaturveränderungen ermitteln können. Solche an sich bekannten Bewegungsmelder sind kostengünstig verfügbar. Sie können vorteilhafterweise einen großen Raumwinkelbereich überwachen. So kann beispielsweise ein definierter Raum über einen ebenen Winkel von 360° rund um das Gerät mit geringem Aufwand erfasst werden. Dadurch können beispielsweise mehrere Geräte in demselben architektonischen Raum unterschiedliche Teilräume überwachen. Auf diese Weise kann vermieden werden, dass mehrere oder alle Geräte gleichzeitig eingeschaltet werden, sobald eine Person den Raum betritt. Dabei können planmäßig auch mehrere überwachte Teilräume überlappen oder übereinstimmen, wenn zu einer bestimmten Tätigkeit ohnehin mehrere Geräte benötigt werden. Generell können mehrere oder gar alle medizinische Geräte in einem Raum mit demselben Personendetektor verbunden sein.

Zugangsberechtigungsmittel wie RFID-Chipkarten können dazu genutzt werden, den Zugriff anhand ihrer Zugangsberechtigungsinformationen auf das medizinische Gerät einzuschränken. So kann, insbesondere unabhängig vom Schalten des Betriebs- oder Ruhezustandes eines Geräteteils, dem Bedienpersonal abhängig von den individuellen Zugangsrechten nur Zugriff auf vorbestimmte Funktionen gewährt werden, beispielsweise Wartungs- oder Reinigungsfunktionen (beispielsweise für nichtmedizinisches Personal), Geräteeinstellungen und Patientendaten sowie Zugriff auf Messwerte und Diagnosedaten (beispielsweise für medizinisches Assistenzpersonal) oder das Auslösen/Durchführen einer diagnostischen Messung und/oder therapeutischen Behandlung (beispielsweise für ärztliches Personal). Auch eine von den Zugangsrechten abhängige sicherheitstechnische Steuerung ist möglich. So kann das Gerät beispielsweise so eingerichtet sein, dass ein ophthalmologischer Laser nur bei Anwesenheit einer Person mit augenärztlichen Zugangsrechten und einer speziellen Laserberechtigung eingeschaltet werden kann. Die Laserberechtigung kann beispielsweise nur an Personen vergeben werden, die im Umgang mit Lasern besonders geschult sind. Alternativ ist es möglich, dass Schutzeinrichtungen, beispielsweise Schutzblenden gegen Laserstrahlung, nur von Personen mit besonderen Zugangsrechten, also beispielsweise gemäß den vorhergehenden Beispiel mit augenärztlichen Zugangsrechten und einer speziellen Laserberechtigung, deaktiviert werden können. Durch beide Alternativen kann ein versehentliches Einschalten des Lasers vermieden werden.

Zu diesem Zweck kann die aus den Zugangsberechtigungsmitteln zu ermittelnde Zugangsberechtigungsinformation entweder eine reine Personenidentifikationsgröße oder unmittelbar einzelne Zugangsrechte umfassen. Bei einer reinen Personenidentifikationsgröße fragt die Steuereinheit die eigentlichen Zugangsrechte der detektierten Person zweckmäßigerweise über eine unabhängige Datenanbindung bei einer zentralen Kontrolleinheit ab. Die zentrale Kontrolleinheit sollte mit einer entsprechenden Datenbank ausgerüstet sein. Die Kontrolle der Zugangsberechtigung durch Vergleich der tatsächlichen Zugangsrechte der detektierten Person mit den notwendigen, funktionsabhängig vorgegebenen Zugangsrechten kann dabei entweder in der Steuereinheit oder in der entfernten Kontrolleinheit durchgeführt werden. Im zweiten Fall übergibt die Steuereinheit bei der Abfrage auch die notwendigen Zugangsrechte für die aktuell vom Bedienpersonal benötigte Funktion an die zentrale Kontrolleinheit.

Es können verschiedene Reichweitenbereiche erfasst werden. Beispielsweise kann ein entfernungsauflösender Beschleunigungssensor mit berührungsempfindlichen Bedienelementen kombiniert werden, um auch bei nahezu bewegungslosem Patienten und Bedienungspersonal eine automatische Abschaltung während einer Untersuchung/Behandlung, die nicht anderweitig ermittelbar ist, zu verhindern.

Die Reaktion des medizinischen Geräts auf die Detektion einer anwesenden oder sich nähernden Person kann mehrstufig ausgestaltet werden. Beispielsweise können auf unterschiedlichen Stufen verschiedene Geräteteile in den Betriebszustand geschaltet werden. Die Geräteteile können dabei von Stufe zu Stufe alternativ oder kumulativ in ihren Betriebszustand geschaltet werden. Die Stufen können beispielsweise in Abhängigkeit von Zugangsberechtigungsinformationen definiert werden. Je nach Zugangsberechtigung der detektierten Person werden dann unterschiedliche Geräteteile oder Geräteteilegruppen in den Betriebszustand geschaltet. Beispielsweise wird für nichtmedizinisches Bedienpersonal nur ein Steuerrechner, nicht aber eine Behandlungseinheit eingeschaltet, so dass Wartungsarbeiten möglich sind. Alternativ oder zusätzlich können die Stufen in Abhängigkeit der Entfernung der Person zum Gerät definiert sein, sofern die Detektion entfernungsaufgelöst möglich ist. So können beispielsweise mit abnehmender Entfernung stufenweise mehr und mehr Geräteteile in den Betriebszustand geschaltet werden. Dazu können Entfernungsbereiche definiert werden. Vorzugsweise werden diejenigen Geräteteile mit der längsten Anlaufzeit zuerst, also bei Detektion im weitesten Entfernungsbereich, und diejenigen mit der kürzesten Anlaufzeit und/oder dem höchsten Energieverbrauch und/oder dem höchsten Verschleiß (insbesondere beim Einschaltvorgang) zuletzt, also bei Detektion im geringsten Entfernungsbereich, in den Betriebszustand geschaltet.

Besonders bevorzugt ist eine Ausprägung der Erfindung, in der das Gerät ein ophthalmologisches Gerät ist, insbesondere eine Spaltlampe, eine Funduskamera, ein Gerät zur Messung von Augeninnenabmessungen oder ein Laser, insbesondere in der refraktiven Chirurgie. Ophtalmologische Geräte enthalten in der Regel eine Hochleistungslichtquelle mit entsprechendem Energieverbrauch. Sie ziehen daher besonderen Vorteil aus der erfindungsgemäßen Verbrauchsreduktion. Im Falle eines Lasers ist die Temperaturstabilisierung ein großer Verbraucher, der von der Erfindung profitieren kann. Wegen der Verletzungsgefahr, die von einem unbeaufsichtigt aktiven Laser ausgehen kann, wird durch die erfindungsgemäße, automatische Aus- und/oder Einschaltung auch die Betriebssicherheit erhöht.

Es ist vorteilhafterweise möglich, dass im Ruhezustand an dem Teilgerät eine reduzierte, von Null verschiedene Betriebsspannung anliegt. So muss das Gerät beziehungsweise das Geräteteil nicht vollständig abgeschaltet werden. Dadurch kann trotz des reduzierten Energieverbrauchs im Ruhezustand die Rückkehr in den Betriebszustand schneller erfolgen. Dieser Ruhezustand mit reduzierter Betriebsspannung kann beispielsweise durch eine Akkumulatorpufferung aufrechterhalten werden.

Vorzugsweise ist an dem medizinischen Gerät ergänzend oder alternativ zu einer zeitbasierten automatischen Schaltung in den Ruhezustand ein Bedienelement zum manuellen Schalten des Geräts vom Betriebszustand in den Ruhezustand vorgesehen. Das Bedienpersonal kann das Gerät damit unmittelbar nach Benutzungsende aktiv in den Ruhezustand versetzen. Ein Zeitraum der Nichtbenutzung braucht so nicht abgewartet zu werden, was den Energieverbrauch reduziert.

Die Steuereinheit und/oder der Personendetektor müssen nicht zwangsläufig in oder an dem medizinischen Gerät angeordnet sein, sondern können sich bei gleicher Funktion entweder separat oder zusammen in einer externen Einheit befinden. Die externen Einheiten können beliebig angeordnet sein, soweit die Position zur Personendetektion geeignet ist.

Beispielsweise kann es sich bei dem Personendetektor um eine am Raumeingang angeordnete Lichtschranke handeln. Vorzugsweise wird eine mehrkanalige Lichtschranke verwendet, so dass zwischen Betreten und Verlassen des Raums unterschieden werden kann. Das Schalten in den Betriebszustand erfolgt dann nur bei Betreten, ein eventuell vorgesehenes Schalten in den Ruhezustand nur bei Verlassen des Raums. Dabei kann eine Einschaltverzögerung vorgesehen werden, so dass das Geräteteil bei einer kurzen Abfolge von Betreten und (Wieder-)Verlassen nicht in den Betriebszustand geschaltet wird. Ebenso kann eine Ausschaltverzögerung vorgesehen werden, so dass das Geräteteil bei einer kurzen Abfolge von Verlassen und (Wieder-)Betreten nicht in den Ruhezustand geschaltet wird.

Nachfolgend wird die Erfindung anhand von Ausführungsbeispielen näher erläutert.

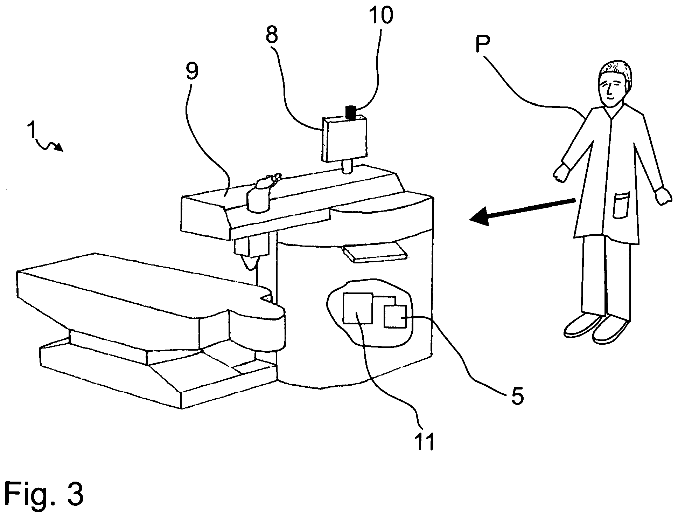

In den Zeichnungen zeigt:
Fig. 3 eine schematische Darstellung einer opthtalmologischen Excimer-Lasers mit Bewegungsmelder.

Fig. 3 zeigt ein medizinisches Gerät 1 in Form eines ophtalmologischen Excimer-Lasers für die refraktive Chirurgie. Das Gerät 1 ist an einem Monitor 8 der Laser- und Bedieneinheit 9 mit einem Bewegungsmelder 10 ausgerüstet, der einen ebenen Erfassungswinkel von 360° aufweist. Der Bewegungsmelder 10 ist mit einer Steuereinheit 5 verbunden, die ihrerseits mit einer Lasersteuerung 11, die insbesondere eine Temperaturstabilisierung (nicht abgebildet) regelt, verbunden ist.

Im beispielhaften Ausgangszustand ist die Laser- und Bedieneinheit 9 in einem Ruhezustand mit geringem Energieverbrauch. Ein integrierter Rechner (nicht dargestellt) befindet sich in einem Schlafmodus. Der Laser (nicht abgebildet) wie auch seine Temperaturstabilisierung sind über die Lasersteuerung 11 ausgeschaltet und somit ebenfalls im Ruhezustand. Eine Gehäuselüftung (nicht abgebildet) ist ausgeschaltet. Der Energieverbrauch, die Abwärmeproduktion und der Lärmpegel des Geräts 1 sind minimal.

Sobald der Bewegungsmelder 10 eine sich bewegende Person P detektiert, schaltet die Steuereinheit 5 alle im Ruhezustand befindlichen Geräteteile über die Lasersteuerung 11 in den Betriebszustand. Dieser wird erst nach einer Anlaufzeit von etwa einer Minute erreicht. Da die Person P bereits beim Betreten des das Gerät 1 umgebenden architektonischen Innenraums berührungslos detektiert wird und in der Regel noch einige Vorbereitungen für eine Behandlung mit dem Gerät 1 trifft, ist das Gerät 1 einsatzbereit noch bevor die Person P es benutzen möchte.

Nach der Benutzung des Geräts verlassen die Person P und der behandelte Patient das Gerät 1. Die Steuereinheit 5 erfasst daraufhin einen Zeitraum von zwanzig Minuten der Nichtbenutzung sowie der Abwesenheit von Personen P, wobei sie bereits nach neunzehn Minuten eine akustische und optische Vorwarnung ausgeben kann. Die Vorwarnung kann vom Bedienpersonal manuell abgeschaltet und zugelassen werden. Die Abschaltung kann als generelle Vorgabe oder im Einzelfall, insbesondere nach Beginn der Ausgabe der Vorwarnung, erfolgen. Nach Ablauf des Zeitraums der Nichtbenutzung schaltet die Steuereinheit 5 alle ruhefähigen Geräteteile wieder in den Ruhezustand für minimalen Energieverbrauch.

In allen Ausführungsformen kann die Steuereinheit 5 mit einer selbstlernenden Zeitsteuerung ausgerüstet oder programmiert werden, welche die Benutzungsgewohnheiten des Bedienpersonals erfasst, speichert und daraus ein zukünftiges Schaltverhalten für das Umschalten aus dem Ruhezustand in den Betriebszustand ableitet. So kann beispielsweise nach einigen Benutzungsdurchgängen abgeleitet werden, dass das Gerät 1 erst bei Anwesenheit von mindestens zwei Personen benutzt wird. Die Steuereinheit 5 wird dann den Betriebszustand des Geräts 1 beziehungsweise der ruhenden Teilgeräte erst einschalten, wenn zwei Personen detektiert worden sind. Ein solches Schaltverhalten kann auch ohne selbstlernende Zeitsteuerung fest in die Steuereinheit 5 integriert werden.

Die Einleitung des Ruhezustands eines oder mehrerer Geräteteile oder die zum Ruhezustand führenden Detektionsdaten können in weiterführenden Ausgestaltungen auch an externe Geräte ausgegeben werden. Auf diese Weise können beispielsweise ein an das Gerät 1 extern angeschlossener Drucker, Netzsteckdosen, eine Fixierleuchte oder ein Instrumententisch in einen Ruhezustand um- oder ausgeschaltet werden.

In allen Ausführungsformen der Erfindung kann eine Kontrolle der Zugangsberechtigung des Bedienpersonals durchgeführt und der Zugriff auf das medizinische Gerät 1 und die darin gespeicherten Daten anhand einer Zugangsberechtigungsinformation der detektierten Personen P gewährt oder eingeschränkt werden. Die personenindividuell vorgebbare Zugangsberechtigungsinformation wird dazu vorzugsweise aus tragbaren Zugangsberechtigungsmitteln vorzugsweise über Nahfunk ermittelt, beispielsweise per RFID. Die daraus zu ermittelnden Zugangsrechte können rollenbasiert und/oder bezüglich Daten auch eigentümerbasiert vergeben werden, wobei beispielsweise durch Unterrechte zwischen rein lesendem Zugriff (Datenabfrage), schreibendem Zugriff (Dateneingabe), Wartungszugriff und Diagnose-/Behandlungszugriff differenziert werden kann. Eine weitere Differenzierung kann zwischen der Art der Daten erfolgen. So können beispielsweise einer Gruppe ("Rolle") "medizinisches Assistenzpersonal" oder auch nur einzelnen Assistenzpersonen lediglich Adressdaten von Patienten zugänglich gemacht werden, nicht aber Messwerte oder Diagnosen. Unter Zugänglichmachen fällt dabei nicht nur die Ausgabe am Bildschirm, sondern auch auf einen Drucker, einen Datenträger oder in Form eines Datentransfers über ein Rechnernetz, beispielsweise ein Intranet einer Arztpraxis oder eines Krankenhauses, zu einem Zentralrechner oder an eine andere Person wie den behandelnden (Haus-)Arzt oder einen externen Spezialisten, beispielsweise per E-Mail. Zweckmäßigerweise können die Zugangsrechte für diese unterschiedlichen Ausgabekanäle differenziert vergeben werden, so dass beispielsweise ein Versand per E-Mail nur einzelnen, ausgewählten Personen möglich ist.

Zusätzlich zur Ermittlung der Zugangsberechtigungsinformation mittels Nahfunk kann am Gerät 1 ein individuelles Passwort abgefragt werden. Enthält die Zugangsberechtigungsinformation eine Personenidentifikationsgröße, so ist damit die Identität der jeweils detektierten Person P bekannt. Bei nicht identifizierbaren Personen P kann stattdessen ein Benutzername abgefragt werden. Zweckmäßigerweise werden sowohl erfolgreiche als auch fehlgeschlagene Passworteingaben aufgezeichnet. Bei Überschreiten einer vorgebbaren Anzahl von fehlgeschlagenen Versuchen kann das Gerät 1 eine Warnmeldung ausgeben und diese insbesondere an eine zentrale Kontrolleinheit übermitteln. Es ist auch denkbar, dass das Gerät 1 bei Überschreiten einer vorgebbaren Anzahl von fehlgeschlagenen Versuchen so in einen Sperrzustand übergeht, dass es nur durch einen Eingriff von Wartungspersonal wieder funktionsfähig wird. Vorzugsweise bietet die Steuereinheit 5 einen Notfallmodus an, in dem auch ohne Passworteingabe und trotz einer eventuellen Sperrung auf Daten und Funktionen des Geräts 1 zugegriffen werden kann. Die Anwendung dieses Notfallmodus sollte zu Überprüfungszwecken aufgezeichnet werden.

Stellt die Steuereinheit 5 einen Zeitraum der Nichtbenutzung fest, insbesondere in Verbindung mit einer Abwesenheit von Personen P, so kann sie sicherheitshalber eine erneute Passworteingabe verlangen, bevor das Gerät 1 wieder benutzt werden kann. Daneben kann die Steuereinheit 5 für eine Fernwartung eingerichtet sein, die vorzugsweise ausschließlich über eine Passwortabfrage und/oder verschlüsselt möglich ist. Jede Fernwartung und alle während einer Fernwartung durchgeführten Handlungen sollten mit Uhrzeit und Benutzeridentifikation protokolliert werden.

Die Zuteilung der Zugangsrechte auf die einzelnen Personen P oder auf Benutzerrollen kann entweder lokal am Gerät 1 oder in einer zentralen Kontrolleinheit erfolgen. Im zweiten Fall können die Zugangsrechte für mehrere oder alle medizinischen Geräte 1 (und auch andere Geräte) beispielsweise einer Arztpraxis, einer Krankenhausabteilung oder eines ganzen Krankenhauses zentral verwaltet werden. Sie sind dadurch nicht lokal am Gerät 1 manipulierbar. Neben der Zugriffsbeschränkung/-gewährung können Lesezugriffe, Änderungen an den vorhandenen Daten oder das Anlegen neuer Daten mit Uhrzeit und Benutzeridentifikation der bedienenden Person P protokolliert werden. Sämtliche nichtlokalen Abfragen oder Datenzugriffe der Steuereinheit 5, insbesondere bei einer zentralen Kontrolleinheit, sollten verschlüsselt erfolgen, um Manipulationen zu verhindern.

### Bezugszeichenliste

- 1: Medizinisches Gerät
- 2: Steuereinheit
- 3: Monitor
- 4: Laser- und Bedieneinheit
- 5: Bewegungsmelder
- 6: Lasersteuerung

- P: Person

## Patentansprüche

1. Medizinisches Gerät (1), bei dem zumindest ein Geräteteil (4, 9, 11) zwischen einem Ruhezustand und einem Betriebszustand umschaltbar ist, aufweisend einen Personendetektor (6, 10) und eine Steuereinheit (5), die, falls der Personendetektor (6, 10) eine entfernt vom Gerät (1) anwesende Person (P) detektiert, zumindest das Geräteteil (4, 9, 11) in den Betriebszustand schaltet, wobei mittels des Personendetektors (6, 10) eine Person (P) detektierbar ist, die weiter vom Gerät (1) entfernt ist als es für eine Benutzung des Geräts (1) erforderlich ist, **dadurch gekennzeichnet, dass** der Personendetektor als Bewegungsmelder (10) ausgebildet ist und in einem bestimmten Raumbereich passiv mittels Infrarotstrahlung Bewegungen und/oder Temperaturveränderungen ermitteln kann, wobei mittels des Personendetektors (6, 10) eine Person (P) detektierbar ist, die mindestens 1 m vom Gerät (1) entfernt ist, wobei die Steuereinheit (5) zunächst ermittelt, ob die detektierte Person (5) sich dem Gerät (1) nähert, und nur bei ermittelter Annäherung in den Betriebszustand schaltet.

2. Medizinisches Gerät (1) nach Anspruch 1, **dadurch gekennzeichnet, dass** die Steuereinheit (5) nach Ablauf eines vorgebbaren oder vorgegebenen Zeitraums, in dem die Steuereinheit (5) keine Benutzung des Geräts (1) ermittelt und/oder in dem der Personendetektor (6, 10) keine Person detektiert, zumindest das Geräteteil (4, 9, 11) vom Betriebszustand in den Ruhezustand schaltet.

3. Medizinisches Gerät (1) nach Anspruch 2, **dadurch gekennzeichnet, dass** die Steuereinheit (5) zu einen vorgebbaren oder vorgegebenen Vorwarnzeitpunkt vor Ablauf des Zeitraums ein Warnsignal ausgibt.

4. Medizinisches Gerät (1) nach einem der Ansprüche 1 bis 3, **dadurch gekennzeichnet, dass** mittels des Personendetektors (6, 10) eine Person (P) in einem das Gerät umgebenden architektonischen Innenraum, in einem Raumbereich im Umfeld des Geräts (1) oder an einer bestimmten Stelle detektierbar ist.

5. Medizinisches (1) Gerät nach einem der vorhergehenden Ansprüche, **dadurch gekennzeichnet, dass** das Gerät (1) ein ophthalmologisches Gerät ist.

6. Medizinisches Gerät (1) nach Anspruch 5, **dadurch gekennzeichnet, dass** das Gerät eine Spaltlampe, eine Funduskamera, ein Gerät zur Messung von Augeninnenabmessungen oder ein Laser ist.

7. Medizinisches Gerät (1) nach Anspruch 6, **dadurch gekennzeichnet, dass** das Geräteteil (4, 9, 11) eine Lichtquelle oder eine Temperaturstabilisierung ist.

8. Medizinisches Gerät (1) nach einem der vorhergehenden Ansprüche, **dadurch gekennzeichnet, dass** im Ruhezustand an dem Geräteteil (4, 9, 11) eine reduzierte, von Null verschiedene Betriebsspannung anliegt.

9. Medizinisches Gerät (1), bei dem zumindest ein Geräteteil (4, 9, 11) elektrisch betrieben und zwischen einem Ruhezustand und einem Betriebszustand umschaltbar ist, nach einem der vorhergehenden Ansprüche, **gekennzeichnet durch** eine Steuereinheit (5), die nach Ablauf eines vorgebbaren oder vorgegebenen Zeitraums, in dem die Steuereinheit (5) keine Benutzung des Geräts (1) ermittelt, zumindest das Geräteteil (4, 9, 11) vom Betriebszustand in den Ruhezustand schaltet.

10. Medizinisches Gerät (1) nach einem der vorhergehenden Ansprüche, **gekennzeichnet durch** ein Bedienelement zum manuellen Schalten des Geräts (1) vom Betriebszustand in den Ruhezustand.

## Claims

1. Medical device (1) in which at least one device part (4, 9, 11) can be changed over between a quiescent state and an operating state, having a person detector (6, 10) and a control unit (5) which, if the person detector (6, 10) detects a person (P) who is present away from the device (1), switches at least the device part (4, 9, 11) to the operating state, wherein a person (P) who is further away from the device (1) than is required for use of the device (1) can be detected by means of the person detector (6, 10), **characterized in that** the person detector is in the form of a motion detector (10) and can passively determine movements and/or temperature changes in a particular spatial region by means of infrared radiation, wherein a person (P) who is at least 1 m away from the device (1) can be detected by means of the person detector (6, 10), wherein the control unit (5) initially determines whether the detected person (5) is approaching the device (1) and switches to the operating state only if approach is determined.

2. Medical device (1) according to Claim 1, **characterized in that** the control unit (5) switches at least the device part (4, 9, 11) from the operating state to the quiescent state after the expiry of a predefinable or predefined period in which the control unit (5) does not determine any use of the device (1) and/or in which the person detector (6, 10) does not detect a person.

3. Medical device (1) according to Claim 2, **characterized in that** the control unit (5) outputs a warning signal at a predefinable or predefined early warning time before expiry of the period.

4. Medical device (1) according to one of Claims 1 to 3, **characterized in that** a person (P) can be detected in an architectural internal space surrounding the device, in a spatial region in the environment of the device (1) or at a particular location by means of the person detector (6, 10).

5. Medical device (1) according to one of the preceding claims, **characterized in that** the device (1) is an ophthalmological device.

6. Medical device (1) according to Claim 5, **characterized in that** the device is a slit lamp, a retinal camera, a device for measuring internal eye dimensions or a laser.

7. Medical device (1) according to Claim 6, **characterized in that** the device part (4, 9, 11) is a light source or a temperature stabilization means.

8. Medical device (1) according to one of the preceding claims, **characterized in that** a reduced operating voltage which differs from zero is applied to the device part (4, 9, 11) in the quiescent state.

9. Medical device (1) in which at least one device part (4, 9, 11) is electrically operated and can be changed over between a quiescent state and an operating state according to one of the preceding claims, **characterized by** a control unit (5) which switches at least the device part (4, 9, 11) from the operating state to the quiescent state after expiry of a predefinable or predefined period in which the control unit (5) does not determine any use of the device (1).

10. Medical device (1) according to one of the preceding claims, **characterized by** an operating element for manually switching the device (1) from the operating state to the quiescent state.

## Revendications

1. Appareil médical (1), avec lequel au moins une partie d'appareil (4, 9, 11) peut être permutée entre un état de repos et un état opérationnel, possédant un détecteur de personne (6, 10) et une unité de commande (5) qui, dans le cas où le détecteur de personne (6, 10) détecte une personne (P) présente à distance de l'appareil (1), commute au moins la partie d'appareil (4, 9, 11) dans l'état opérationnel, une personne (P) pouvant être détectée au moyen du détecteur de personne (6, 10), laquelle est plus éloignée de l'appareil (1) que ce qui est nécessaire pour une utilisation de l'appareil (1), **caractérisé en ce que** le détecteur de personne est réalisé sous la forme d'un détecteur de mouvement (10) et peut déterminer passivement des mouvements et/ou des variations de température dans une zone d'espace donnée au moyen d'un rayonnement infrarouge, le détecteur de personne (6, 10) permettant de détecter une personne (P) qui est éloignée d'au moins 1 m de l'appareil (1), l'unité de commande (5) déterminant tout d'abord si la personne (5) détectée s'approche de l'appareil (1) et ne commutant dans l'état opérationnel que dans le cas de la détermination d'une approche.

2. Appareil médical (1) selon la revendication 1, **caractérisé en ce que** l'unité de commande (5), après l'écoulement d'une période pouvant être prédéfinie ou qui est prédéfinie pendant laquelle l'unité de commande (5) ne détermine aucune utilisation de l'appareil (1) et/ou pendant laquelle le détecteur de personne (6, 10) ne détecte aucune personne, commute au moins la partie d'appareil (4, 9, 11) de l'état opérationnel à l'état de repos.

3. Appareil médical (1) selon la revendication 2, **caractérisé en ce que** l'unité de commande (5) délivre un signal d'alerte à un instant de préalerte pouvant être prédéfini ou qui est prédéfini avant l'écoulement de la période.

4. Appareil médical (1) selon l'une des revendications 1 à 3, **caractérisé en ce que** le détecteur de personne (6, 10) permet de détecter une personne (P) dans un espace intérieur architectonique qui entoure l'appareil, dans une zone d'espace dans l'environnement de l'appareil (1) ou à un endroit défini.

5. Appareil médical (1) selon l'une des revendications précédentes, **caractérisé en ce que** l'appareil (1) est un appareil ophtalmologique.

6. Appareil médical (1) selon la revendication 5, **caractérisé en ce que** l'appareil est une lampe à fente, un rétinographe, un appareil servant à mesurer les dimensions intérieures de l'oeil ou un laser.

7. Appareil médical (1) selon la revendication 6, **caractérisé en ce que** la partie d'appareil (4, 9, 11) est une source de température ou un système de stabilisation de la température.

8. Appareil médical (1) selon l'une des revendications précédentes, **caractérisé en ce que** dans l'état de repos, une tension de service réduite, différente de zéro, est appliquée à la partie d'appareil (4, 9, 11).

9. Appareil médical (1), avec lequel au moins une partie d'appareil (4, 9, 11) est à fonctionnement électrique et peut être permutée entre un état de repos et un état opérationnel, selon l'une des revendications précédentes, **caractérisé par** une unité de commande (5) qui, après l'écoulement d'une période pouvant être prédéfinie ou qui est prédéfinie pendant laquelle l'unité de commande (5) ne détermine aucune utilisation de l'appareil (1), commute l'au moins une partie d'appareil (4, 9, 11) de l'état opérationnel à l'état de repos.

10. Appareil médical (1) selon l'une des revendications précédentes, **caractérisé par** un élément de commande destiné à commuter manuellement l'appareil (1) de l'état opérationnel à l'état de repos.
